# EUROPEAN PATENT SPECIFICATION

(11) **EP 1 677 760 B1**
(45) Date of publication and mention of the grant of the patent: **02.01.2013**
(21) Application number: 04795818.6
(22) Date of filing: 21.10.2004
(51) Int. Cl.: A61K 9/10, A61K 31/167

(54) **CONTROLLED RELEASE ANALGESIC SUSPENSIONS**
ANALGETISCHE SUSPENSIONEN MIT KONTROLLIERTER FREISETZUNG
SUSPENSIONS ANALGESIQUES A LIBERATION CONTROLEE

(30) Priority: 30.10.2003 US 697840
(43) Date of publication of application: 12.07.2006
(73) Proprietor: McNeil-PPC, Inc., Skillman, NJ 08558-9418 (US)
(72) Inventor: WYNN, David, W., Huntingdon Valley, Pennsylvania 19006 (US); MCNALLY, Gerard, Berwyn, Pennsylvania 19312 (US); PARIKH, Nick, Long Valley, New Jersey 07853 (US)
(74) Representative: Mercer, Christopher Paul
(86) International application number: PCT/US2004/034708
(87) International publication number: WO 2005/044246

(56) References cited:
- EP-A- 0 717 992
- US-A- 4 980 170
- US-A- 6 126 967
- US-A- 6 126 969
- US-B1- 6 254 891

## Description

The present invention relates to a controlled release pharmaceutical formulation suitable for liquid dosage forms for the administration of active ingredients, such as analgesics.

### Background of the Invention

Therapeutic agents for treating pain, inflammation, and fever include analgesics, anti-inflammatories, and antipyretics. Non-steroidal anti-inflammatory drugs (NSAID's) are one type of such therapeutic agents. They include propionic acid derivatives, acetic acid derivatives, fenamic acid derivatives, biphenylcarbodylic acid derivatives, oxicams, and cyclooxygenase-2 (COX-2) selective NSAID's.

Propionic acids include for example ibuprofen, naproxen, and ketoprofen. Ibuprofen in particular is a widely used, well known NSAID possessing analgesic and antipyretic properties. It has been commercially available as an over-the-counter drug in many forms for several years. Ibuprofen is chemically known as 2-(4-isobutylphenyl)-propionic acid.

NSAID's are typically administered on a once to four times daily basis, with the daily dose ranging from about 50 to about 2000 milligrams, preferably from about 100 to 1600 and most preferably from about 200 to about 1200 milligrams.

Acetaminophen is a well-known analgesic, with a daily dose ranging from about 325 to about 4000 m illigrams, preferably from about 650 to about 4000 milligrams. Acetaminophen was first used in medicine by Van Mering in 1893, but only since 1949 has it gained in popularity as an effective alternative to aspirin for analgesic uses in the over the counter market. The pharmacology of APAP is reviewed by B. Ameer et al., Ann. Int. Med. 87, 202 (1977). Considering the widespread use of APAP and the volume of its manufacture, both its manufacture and its use as an analgesic are well known to persons skilled in the art.

It is known to administer NSAID's, acetaminophen, and other drugs in multiple doses over 12 or 24 hours. For example, it is known to administer multiple doses containing equal amounts of ibuprofen over 12 to 24 hours. Additionally, it is also known to administer a higher initial dose, followed by relatively low maintenance doses. See, e.g., Palmisano et al., Advances in Therapy, Vol. 5, No. 4, July/August 1988 (use of multiple doses of ketoprofen (initial dose of 150 mg followed by subsequent doses of 75 mg) and ibuprofen (initial dose of 800 mg followed by subsequent doses of 400 mg)).

In particular, with respect to orally-administered analgesics, the desire to extend the duration of the therapeutic effect led to the development of controlled release formulations that enable a single daily administration. Beneficially, once-daily administration improves patient compliance with recommended dosages during therapeutic treatment.

Controlled release pharmaceutical dosage forms have long been used to optim ize drug delivery and enhance patient compliance, especially by reducing the number of doses of medicine the patient must take in a day. For this purpose, it is often desirable to reduce the rate of release of a drug or other active ingredient from a dosage form into the gastro-intestinal ("g.i.") fluids of a patient, especially in order to provide prolonged action of the drug in the body.

The rate at which an orally delivered drug reaches its site of action in the body depends on a number of factors, including the rate and extent of drug absorption into the blood through the g.i. mucosa. However, before a drug can be absorbed into the blood, it must first be dissolved in the g.i. fluids. For many drugs, absorption across the g.i. membranes is relatively rapid compared to their dissolution in the g.i. fluids, which thereby renders the dissolution of the drug as the rate limiting step in drug absorption. Therefore, a formulator may effectively control the rate of drug absorption into the blood by modifying the drug's rate of dissolution.

It is also particularly desirable for a pharmaceutical dosage form to deliver more than one drug, each at a modified rate. Because the onset and duration of the therapeutic efficacy of drugs vary widely, as do their respective absorption, distribution, metabolism, and elimination, it is often desirable to modify the release of different drugs in different ways, or to have a first drug immediately released from the dosage form, while a second drug is released in a "modified" manner, e.g., either delayed or controlled.

Well known mechanisms by which a dosage form can deliver a drug at a controlled rate (e.g. sustained, prolonged, extended or retarded release) include diffusion, erosion, and osmosis. It is often practical to design dosage forms that use a combination of the above mechanisms to achieve a particularly desirable controlled release profile for a particular active ingredient.

Disadvantageously, many controlled release applications employ solid dosage units having a final large size and weight. The administration of such dosage units presents a problem especially to those patients with difficulty swallowing, such as children and the elderly. Therefore, it is further desirable to provide such controlled release medicines either in a chewable or orally disintegratable solid form or a liquid form. For many patients, liquid oral dosage forms are more preferred because they can be swallowed without the additional step of chewing.

Oral liquid forms have been commonly used to deliver immediately released medications for many years. See, e.g., U.S. Patent Nos. 5,374,659; 4,788,220; 4,975,465; and 5,183,829. However, the incorporation of a controlled release medication into a liquid dosage form presents significant formulation challenges. In particular, coated or chemically bonded particles are typically employed to carry the modified release portion of the drug. The properties of such particles, as well as those of the liquid vehicle for suspending them, must be compatible so that the particles can be maintained in a uniformly dispersed state. A particular challenge is the prevention of a premature release of drug from the suspended particles during the storage life of the liquid dosage form prior to ingestion by a patient. Additionally, the maintenance of the desired dissolution profile as well as the desired dose uniformity of the liquid dosage form throughout its shelf-life are additional challenges to be addressed In formulating an oral, liquid controlled release suspension product.

In United States Patent No. 5,527,545, active ingredient microgranules were coated with four sequential coatings in order to preserve the release characteristics of the dosage form in a liquid suspension. However, not only did the multiple coating step increase the overall cost and product cycle time of the product, but also the resulting dosage forms failed to provide an immediate release dose to the user.

Therefore, It would be further desirable to have a liquid, controlled release dosage form having a suspendable active ingredient, such as an analgesic, which is not only palatable, but is also In a stable form that guarantees the required release profile after administration. It would further be desirable to have such an analgesic suspension product that provided both an immediate release dose and a sustained release dose of analgesic to the user.

### Summary ot the Invention

The invention is described in the appended claims. Also disclosed is a pharmaceutical dosage form suitable for the administration of NSAIDS and/or acetaminophen in a liquid suspension, said dosage form comprising, consisting of, and/or consisting essentially of:
a) a first portion containing an NSAID and/or acetaminophen, the NSAID and/or acetaminophen being released from the dosage form in a substantially Immediate manner upon contact of the dosage form with a dissolution medium; and
b) a second portion of particles containing NSAID and/or acetaminophen, the NSAID and/or acetaminophen being released from the particles in a controlled manner upon contact of the dosage form with the dissolution medium,
wherein the pharmaceutical dosage form has a duration of therapeutic effect for at least about 8 hours after its administration.

Another embodiment of the present disclosure is directed to a liquid suspension dosage form comprising, consisting of, and/or consisting essentially of :
a) a first portion containing an NSAID and/or acetaminophen, the NSAID and/or acetaminophen being released from the dosage form In a substantially immediate manner upon contact of the dosage form with a dissolution medium;
b) a second portion of particles containing NSAID and/or acetaminophen, the NSAID and/or acetaminophen being released from the particles In a controlled manner upon contact of the dosage form with the dissolution medium; and
c) water, or mixtures of water and a pharmaceutically acceptable water-miscible co-solvent selected from the group consisting of glycols, alcohols, and glycerol,
wherein the dosage form has a duration of therapeutic effect for at least about 12 hours after administration.

Another embodiment of the present disclosure is directed to a method of administering acetaminophen and/or an NSAID In a pharmaceutical dosage form to a mammal in need thereof, said method comprises, consists of, and/or consists essentially of providing to a mammal the dosage form such that the mammal receives an immediate release dose of said acetaminophen and/or NSAID at the beginning of said 12 hour time period, and a controlled release dose of the acetaminophen and/or NSAID over a period of about 12 hours after administration of the dosage form, wherein no further acetaminophen and/or NSAID is provided during said 12 hour time period.

Another embodiment of the present disclosure is directed to a pharmaceutical liquid suspension dosage form comprising, consisting of, and/or consisting essentially of:
particles of an NSAID and/or acetaminophen, the particles being substantially
covered with one layer of a controlled release composition,
wherein the pharmaceutical liquid suspension dosage form has a duration of therapeutic effect for at least about 8 hours after its initial adminstration to a mammal.

Another embodiment of the present invention is directed to a pharmaceutical liquid suspension dosage form comprising, consisting of, and/or consisting essentially of:
a) particles containing NSAID and/or acetaminophen, the particles being substantially covered with one layer of a controlled release coating; and
b) water, or mixtures of water and a pharmaceutically acceptable water-miscible co-solvent selected from the group consisting of glycols, alcohols, and glycerol,
wherein the pharmaceutical dosage form has a duration of therapeutic effect for at least about 8 hours after its administration.

Another embodiment of the present disclosure is directed to a method of administering acetaminophen and/or an NSAID in a liquid suspension pharmaceutical dosage form to a mammal In need thereof, the method comprises, consists of, and/or consists essentially of providing to a mammal said dosage form such that the mammal receives a controlled release dose of said acetaminophen and/or NSAID over a period of about 12 hours after administration of said dosage form, wherein no further acetaminophen and/or NSAID is provided during said 12 hour time period.

Another embodiment of the present disclosure is directed to a pharmaceutical liquid suspension dosage form comprising, consisting of, and/or consisting essentially of :
a) particles containing NSAID and/or acetaminophen, the particles being substantially covered with one layer of a controlled release composition, the controlled release composition being comprised of, based upon the total weight of the controlled release composition, from greater than about 0 percent and less than about 90 percent of an insoluble film forming polymer and greater than about 0 percent to less than about 10 percent of an enteric polymer; and
b) water, or mixtures of water and a pharmaceutically acceptable water-miscible co-solvent selected from the group consisting of glycols, alcohols, and glycerol,
wherein the pharmaceutical dosage form has a duration of therapeutic effect for at least about 12 hours after its administration.

### Brief Description of the Drawings

Figure 1 depicts a graph of active ingredient released (mg) versus time (hours) for a liquid suspension dosage form containing both an immediate dose and a controlled release dose of ibuprofen.
Figure 2 depicts a graph of active ingredient released (mg) versus time (hours) for a liquid suspension dosage form containing only a controlled release dose of ibuprofen.

### Detailed Description of the Invention

As used herein, the term "substantially covers" or "substantially continuous" means that the coating is generally continuous and generally covers the entire surface of the core or underlying layer, so that little to none of the active ingredient or underlying layer is exposed.

As used herein, "ATDAIRD" shall mean the average therapeutic duration of action of an effective immediate release dose" of a particular active ingredient. For example, the typical duration of action, i.e. period of therapeutic effect, of an immediate release dose of ibuprofen or ketoprofen is about 4 to about 6 hours. Accordingly, the ATDAIRD for ibuprofen or ketoprofen is 5 hours. The typical duration of action of an immediate release dose of naproxen is about 8 to about 12 hours. The ATDAIRD for naproxen, therefore is 10 hours. The therapeutic duration of action of a particular active ingredient can readily be determined from the dosing instructions in the labeling for immediate release products containing that particular active ingredient.

As used herein, "modified release" shall apply to the altered release or dissolution of an active ingredient in a dissolution medium, such as g.i. fluids. The active ingredient or ingredients that may be released in a modified manner may be contained within, for example, dosage forms, coatings, or particles, or in any portion thereof, such as, for example, particles dispersed throughout a liquid suspending medium. Types of modified release include: 1) controlled release; or 2) delayed release. By "controlled release," it is mean that, after administration, an active ingredient is released from the dosage form in a substantially continuous, regulated manner, and the the for complete release, i.e. depletion, of the active ingredient from the dosage form is longer than that associated with an immediate release dosage form of the same. Types of controlled release include prolonged, sustained, extended, and the like. By "delayed release," It is meant that, after administration, there is at least one period of time when an active ingredient is not being released from the dosage form.

As used herein, "dissolution medium" shall mean any suitable liquid environment In which the suspension dosage form of the present invention can be dissolved, such as, for example, the In vitro dissolution media used for testing of the product, or gastro-intestinal fluids. Suitable In vitro dissolution media used for testing the dissolution of the active ingredient or ingredients from the suspension dosage form of the present invention include those described on page 786 of USP 23 (1995).

One embodiment of the present disclosure is directed to a controlled release pharmaceutical dosage form suitable for the administration of active ingredients in a liquid suspension containing: a) an immediate release portion, e.g., a portion containing at least one active ingredient that is immediately released from the dosage form; and b) a controlled release portion, e.g. a portion containing at least one active ingredient that is released into the bloodstream in a substantially continuous manner over a controlled period of time such as, for example, from about 4 hours to about 12 hours after initial administration of the dosage form. As used herein, "immediate release" means that the dissolution characteristics of at least one active ingredient meets USP specifications for immediate release tablets containing that active ingredient. For example, for acetaminophen tablets, USP 24 specifies that h pH 5.8 phosphate buffer, using USP apparatus 2 (paddles) at 50 rpm, at least 80% of the acetaminophen contained in the dosage form is released therefrom within 30 minutes after dosing, and for ibuprofen tablets, USP 24 specifies that in pH 7.2 phosphate buffer, using USP apparatus 2 (paddles) at 50 rpm, at least 80% of the ibuprofen contained in the dosage form is released therefrom within 60 minutes after dosing, See USP 24, 2000 Version, 19 - 20 and 856 (1999). Additionally, Ibuprofen suspension may be analyzed for dissolution using pH 5.6 acetate buffer using USP apparatus 2 (paddles) at 50 rpm, whore at least 80% of the Ibuprofen contained In the dosage form is released therefrom within 60 minutes after dosing for an Immediate release dose.

The immediate release portion may contain one or more active ingredients that are dispersed at the molecular level, e.g. melted or dissolved, within the dosage form, or the active ingredient may be In the form of particles, which in turn may be coated or uncoated. In embodiments wherein the active ingredient is in form of particles, the particles (whether , coated or uncoated) typically have an average particles size of from about 1 micron to about 2000 microns. In one embodiment, such particles are in the form of crystals having an average particle size of about 1 micron to about 300 microns. In another embodiment, the particles are In the form of granules or pellets having en average particle size of about 25 microns to about 2000 microns, for example, from about 25 microns to about 1000 microns or from about 26 microns to about 400 microns.

The controlled release portion contains at least one active ingredient In a multiplicity of particles having controlled release properties. In one embodiment, the core of these particles In the controller release portion may be comprised of the active ingredient in a pure, crystalline form, which is substantially coated with a controlled release composition. Alternatively, the particle cores may be comprised of a mixture of granules comprised of one or more active Ingredients with optional ingredients, such as binders, excipients and the like known in the art and such granules are also substantially coated with a controlled release composition. In another embodiment, the active Ingredient particles may be dispersed throughout a matrix comprised of a controlled release composition. In yet another embodiment, one or more active Ingredients may be chemically bound or "complexed" to a resin, e.g. an Ion exchange resin, to form particles, which may optionally be substantially coated with a controlled release coating. As used herein, "substantially coated" shall mean that less than about 1%, e.g. less than about 0.1% of the surface area of the particle is exposed, e.g. not covered, with a desired coating.

One skilled In the art would readily appreciate without undue experimentation that the particular ion exchange resin for use in this embodiment is dependent upon several factors such as, for example, the ionic charge of the active ingredient. An example of a suitable ion exchange resin for NSAID active ingredients includes, but is not limited to, cholestyramine, which is commercially available from Rohm & Haas under the tradename. "Duolite® AP143." Additional details of complexation with polymeric resins are well known in the art and disclosed in, for example, U.S. Patent Nos. 4,221,778; 4,847,077 and 0,001,392.

In one particular embodiment, the controlled release portion of the dosage form is substantially free of ion exchange resins, By "substantially free of ion exchange resins," it is meant that the amount of ion exchange resin, based upon the total weight of all active ingredient particles in the dosage form, is less than about 1 percent, e.g., less than about 0.5 percent or less than about 0.1 percent.

The one or more coating layers on the particles may be comprised of any suitable controlled release compositions as set out in the claims. An example of a suitable controlled release composition Is comprised of, based upon the total weight of the controlled release composition, from about greater than about 0 percent to about 90 percent, e.g. from about 10 percent to about 60 percent, of an insoluble film forming polymer and greater than about 0 percent and less than about 10 percent, e.g. from about 0.6 percent to about 20 percent, of an enteric polymer. The weight ratio of enteric polymer to insoluble film forming polymer in the controlled release composition may be In the range of from about 0.5:99.5 to about 20:80, e.g. from about 5:95 to about 10:90. Similar controlled release compositions may also be used in the matrix throughout which active Ingredient particles may be dispersed.

Suitable insoluble film forming polymers include, but are not limited to, cellulose acetate, ethylcellulose, poly(ethyl acrylate, methyl methacrylate, trimethylammonioethyl methacrylate chloride) 1:2:0.1, which is commercially available from Rohm Pharma under the tradename, "EUDRAGIT RS," and copolymer and mixtures thereof. In one embodiment, the insoluble film forming polymer is selected from cellulose acetate and/or ethylcellulose.

Suitable enteric polymers include, but are not limited to, hydroxypropyl methylcellulose phthalate, hydroxypropyl methylcellulose acetate succinate, cellulose acetate phthalate polyvinylacetate phthalate, polymethacrylate-based polymers, and copolymers and mixtures thereof. Examples of suitable polymethacrylate-based polymers Include, but are not limited to poly(methacryic acid, methyl methacrylate) 1:2, which Is commercially available from Rohm Pharma GmbH under the tradename, "EUDRAGIT S" polymers, and poly(methacrylic acid, methyl methacrylate) 1:1, which is commercially available from Rohm Pharma GmbH under the tradename, "EUDRAGIT L" polymers. In one embodiment, the enteric polymer is selected from non-acrylate compounds, such as hydroxypropyl methylcellulose phthalate, hydroxypropyl methylcellulose acetate succinate, cellulose acetate phthalale, polyvinylacetate phthalate, and copolymers and mixtures thereof.

In one embodiment, the controlled release composition contains, based upon the total weight of the controlled release composition, less than about 1 percent or less than about 0.25 percent of enteric polymers.

The active ingredient particles coated with a controlled released composition contain, based upon the total dry weight of such coated particles, from about 5 percent to about 40 percent, e.g. from about 10 percent to about 30 percent of the controlled release composition In the form of at least one coating layer.

The coated active ingredient particles may be formed by any suitable method known In the art. Suitable particle forming and coating methods include high sheer granulation, fluid bed granulation, e.g. rotor granulation, fluid bed coating, coaccervation, spray drying, spray congealing, and the like and are described for example In Pharmaceutical Dosage Forms; Tablets Volume 3, edited by Herbert A. Lieberman and Leon Lachman, Chapters 2, 3, and 4 (1982). In one embodiment, the average diameter of the particles coated with a controlled release composition is from about 20 to about 400 microns, e.g. from about 50 microns to about 300 microns.

The dosage form of the present invention contains one active ingredient according to claim 1 or additionally more active Ingredients. Suitable active Ingredients broadly include, for example, pharmaceuticals, minerals, vitamins and other nutraceuticals, oral care agents, flavorants and mixtures thereof. Suitable pharmaceuticals include analgesics, anti-inflammatory agents, antiarthritics, anesthetics, antihistamines, antitussives, antibiotics, anti-infective agents, antivirals, anticoagulants, antidepressants, antidiabetic agents, antiemetics, antiflatulents, antifungals, antispasmodics, appetite suppressants, bronchodilators, cardiovascular agents, central nervous system agents, central nervous system stimulants, decongestants, oral contraceptives, diuretics, expectorants, gastrointestinal agents, migraine preparations, motion sickness products, mucolytics, muscle relaxants, osteoporosis preparations, polydimethylsiloxanes, respiratory agents, sleep-aids, urinary tract agents and mixtures thereof.

Suitable flavorants include menthol, peppermint, mint flavors, fruit flavors, chocolate, vanilla, bubblegum flavors, coffee flavors, liqueur flavors and combinations and the like.

Example of suitable gastrointestinal agents include antacids such as calcium carbonate, magnesium hydroxide, magnesium oxide, magnesium carbonate, aluminum hydroxide, sodium bicarbonate, dihydroxyaluminum sodium carbonate; stimulant laxatives, such as bisacodyl, casoera sagrada, danthron, senna, phenolphthalein, aloe, castor oil, ricinoleic acid, and dehydrocholic acid, and mixtures thereof; H2 receptor antagonists, such as famotadine, ranitidine, cimetadine, nizatidine; proton pump inhibitors such as omeprazole or lansoprazole; gastrointestinal cytoprotectives, such as sucraflate and misoprostol; gastrointestinal prokinetics, such as prucalopride, antibiotics for H. pylori, such as clarithromycin, amoxicillin, tetracycline, and metronidazole; antidiarrheals, such as diphenoxylate and loperamide; glycopyrrolate; antiemetics, such as ondansetron, analgesics, such as mesalamine.

Examples of suitable polydimethylsiloxanes, which include, but are not limited to dimethicone and simethicone, are those disclosed in United States Patent Nos. 4,606,478, 5,275,822, and 6,103,260. As used herein, the term "simethicone" refers to the broader class of polydimethylsiloxanes, including but not limited to simethicone and dimethicone.

Examples of active ingredients are bisacodyl, famotadine, ranitidine, cimetidine, prucalopride, diphenoxylate, loperamide, lactase, mesalamine, bismuth, antacids, and pharmaceutically acceptable salts, esters, Isomers, and mixtures thereof.

Examples of active ingredients are analgesics, anti-inflammatories, and antipyretics, e.g. non-steroidal anti-inflammatory drugs (NSAIDs), including a) propionic acid derivatives, e.g. Ibuprofen, naproxen, ketoprofen and the like; b) acetic acid derivatives, e.g. Indomethacin, diclofenac, sulindac, tolmetin, and the like; o) fenamic acid derivatives, e.g. mefenamic acid, meclofenamic acid, flufenamic acid, and the like; d) biphenylcarboxylic acid derivatives, e.g. diflunisal, flufenisal, and the like; e) oxicams, e.g. piroxicam, sudoxicam, isoxicam, meloxicam, and the like; f) cyclooxygenase-2 (COX-2) selective NSAIDs; and g) pharmaceutically acceptable salts of the foregoing.

Examples of active ingredients are propionic acid derivative NSAID. which are pharmaceutically acceptable analgesics/non-steroidal anti-Inflammatory drugs having a free -CH(CH₃)COOH or -CH₂CH₂COOH or a pharmaceutically acceptable salt group, such as -CH(CH₃)COO-Na+ or CH₂CH₂COO-Na+, which are typically attached directly or via a carbonyl functionality to a ring system, preferably an aromatic ring system.

Examples of useful propionic acid derivatives include ibuprofen, naproxen, benoxaprofen, naproxen sodium, fenbufen, flurbiprofen, fenoprofen, fenbuprofen, ketoprofen, indoprofen, pirprofen, carpofen, oxaprofen, pranoprofen, microprofen, tioxaprofen, suprofen, alminoprofen, tiaprofenic acid, fluprofen, bucloxic acid, and pharmaceutically acceptable salts, derivatives, and combinations thereof.

In the invention, the propionic acid derivative is ibuprofen, 2-(4-isobutylphenyl) propionic acid, or a pharmaceutically acceptable salt thereof, such as the arginine, lysine, or histidine salt of ibuprofen. Other pharmaceutically acceptable salts of ibuprofen are described in US Patent Nos. 4,279,926, 4,873,231, 5,424,075 and 5,510,385.

Examples of active ingredients are acetaminophen, acetyl salicylic acid, ibuprofen, naproxen, ketoprofen, flurbiprofen, diolofenac, cyclobenzaprine, meloxicam, rofecoxib, celecoxib, and pharmaceutically acceptable salts, esters, isomers, and mixtures thereof.

Examples of active ingredients are pseudoephedrine, phenylpropanolamine, chlorpheniramine, dextromethorphen, diphenhydramine, astemizole, terfenadine, fexofenadine, loratedine, desioratadine, cetirizine, mixtures thereof and pharmaceutically acceptable salts, esters, isomers, and mixtures thereof.

The therapeutically effective amount of the active ingredient or ingredients may be present in a "unit dose volume", which can be in the form of a powder or an aqueous suspension. "Therapeutically effective amount," as used wherein, Is an amount of active ingredient that produces the desired therapeutic response upon oral administration. One skilled in the art can readily determine the "therapeutically effective amount" of an active ingredient for a given patient by considering factors such as, for example, the particular active ingredient being administered; the bioavailability characteristics of the active ingredient; the dose regimen desired; the age and weight of the patent; and the like. As used herein, a "unit dose volume" may be any convenient volume for orally administering a dose of a given product to a patient.

In this embodiment, the "unit dose volume" is typically accompanied by dosing directions, which instruct the patient to take an amount of the active ingredient that may be a multiple of the unit dose volume depending on, e.g., the age or weight of the patient. Typically the unit dose volume of the suspension will contain an amount of active ingredient that is therapeutically effective for the smallest patient. For example, suitable unit dose volumes may include one teaspoonful (about 5 mL), one tablespoonful (about 15 mL), one dropper, or one milliliter.

According to the invention, the dosage form may be provided to a mammal in need of treatment, in particular pain relief treatment, in a single administration that provides for the release of the active ingredient in the blood over an extended time period, e.g over about an 8 hour or about a 12 hour period. At time zero, an initial dose of the active ingredient is provided, i.e. administered, to the mammal via of the active ingredient(s) in the immediate release dose portion. The active ingredient continues to be released into the blood after about four, e.g., i.e., about eight, ten, or twelve, hours from initial administration of the formulation containing the active ingredient via the active ingredient(s) in the controlled release dose portion. In other words, the formulation still retains undissolved active ingredient after about four, e.g., i.e., about eight, ten, or twelve, hours from initial administration.

In practicing the present invention, the dosage form may be comprised of, based upon the total weight of the active ingredient, from about 25 percent to about 75 percent of an immediate release dose portion of the active ingredient; and from about 75 percent to about 25 percent of a controlled release dose portion of the active ingredient. The immediate release dose portion and the controlled release dose portion may be combined with a vehicle to form either a dry mixture that can be suspended extemporaneously when needed, or a ready-to-use liquid suspension.

Suitable constituents of the vehicle can include, without limitation, structuring agents; swelling agents; surfactants; sugars; buffering substances such as citric acid and sodium citrate; glycine and hydrochloric acid, sodium phosphate, and potassium phosphate; preservatives and bacteriostatic agents such as esters of p-hydroxybenzoic acid; colorants; and various flavorings and sweeteners commonly used in pharmaceuticals.

Examples of suitable sweeteners include, but are not limited to any known sweetening agent such as sugars, sugar alcohols, high intensity sweeteners, and mixtures thereof. Suitable sugars include, but are not limited to sucrose, dextrose, high fructose corn syrup, and maltose. Suitable sugar alcohols include, but are not limited to sorbitol, xylitol, and mannitol. Suitable high intensity sweeteners include, but are not limited to sucralose, aspartame, saccharin, and acesulfame K.

In the invention effective amount of a buffering agent is used In order to have the pKa of NSAiD active ingredient contained In the controlled release portion of the liquid suspension dosage form be greater than the pH of the overall liquid suspension dosage form.

In addition, the vehicle may also be comprised of water, or mixtures of water and a pharmaceutically acceptable water-miscible co-solvent known in the art such as, for example, glycols, alcohols and glycerol.

In certain embodiments the dosage form may include any suspending systems known In the art, such as those that typically include one or more structuring agents and/or one or more swelling agents, in one embodiment the dosage form contains, based upon the total weigh of the liquid suspension dosage form, from about 0.1 percent to about 10 percent, of a suspending system. Suitable suspending systems include those disclosed in, for example, United States Patent Nos. 5,374,659, 5,621,005, and 6,409,907.

Structuring agents that are suitable for use in the present invention include hydrophilic polymers such as hydrocolloids. Examples of suitable hydrocolloids include signates, agar, guar gum, locust bean, carrageenan, tara, gum arabic, tragaoanth, pectin, xanthan, gellan, maltodextrin, galactomannan, pusatulan, laminarin, scleroglucan, gum arabic, inulin, karaya, whelan, rhamsan, zooglan, methylan, chitin, cyclodextrin, ohitosan, and combinations thereof. In certain embodiments of the present invention, xanthan gum is the structuring agent.

Xanthan gum is a high molecular weight natural carbohydrate, specifically, a polysaccharide. One xanthan gum that is suitable for use In the present invention is a high molecular weight polysaccharide produced by Xanthomonas campestris. Techniques and strains for producing this polysaccharide are described In U.S. Patent Nos. 4,752,580 and 3,485,719 in one embodiment, the xanthan gum may have a viscosity In a one percent salt solution of from about 1000 to about 1700 cP (mPs-seo), as measured at 25 °C with an LV model Brookfield Synchro-Lectric viscometer at 60 rpm, no. 3 spindle. Suitable xanthan gums are available from, for example, CP Kelco, under the tradename, "Keltrol, "Keltrol TF," and "Keltrol 1000,"

A swelling agent, when exposed to an appropriate aqueous environment, expands without forming a network system. Pregelatinized starch is a particularly good swelling agent. Pregelatinized starch, also known as "instantized" starch, Is precooked so that it swells and begins to thicken instantly when added to cold water. One particularly suitable pregelatinized starch is prepared from modified, stabilized end waxy, maize food starch, and is commercially available from National Starch Company as "INSTAMT STARCH. ULTRASPERSE-M." Other suitable swelling agents include, but are not limited to microcrystalline cellulose and/or hydroxypropylmethylcellulose.

In one embodiment, the suspending system is comprised of a xanthan gum structuring agent with a pregelatinized starch swelling agent. In another embodiment, the suspending system is comprised of, based upon the total weight of the liquid suspension dosage form, from about 0.01 percent to about 1 percent or from about 0.05 percent to about 0.40 percent of xanthan gum and from about 1 percent to about 10 percent or from about 0.5 percent to about 3.0 percent of a pregelatinized starch such as that commercially available from National Starch Company under the tradename, "INSTANT STARCH, ULTRASPERSEM".

The dosage form is in the form of an aqueous pharmaceutical suspension composition and is comprised of, based upon the total weight of active ingredient per volume (w/v or g /100ml) of the aqueous pharmaceutical suspension, from greater than about 0 percent to about 40 percent, e.g. about 0.05 percent to about 0.2 percent, or about 1.6 percent to about 10 percent, or about 15 percent to about 40 percent of at least one active ingredient.

The active ingredient is ibuprofen, the amount of active ingredient in the suspension dosage form is, based upon the total weight of active ingredient per volume (w/v) of the aqueous suspension dosage form, from about 50 to about 200 mg, e.g. from about 50 mg to about 100 mg per teaspoonful of aqueous suspension dosage form, or is about 40 mg of active ingredient per 1 mL of the aqueous suspension dosage form, which is equivalent to, based upon the total weight of active ingredient per volume (w/v) of the aqueous suspension dosage form, from about 1 percent to about 4 percent.

One embodiment of the present invention is directed to a liquid measurable suspension composition that includes, based upon the total weight of the suspension: a) from about 0.05 percent to about 40 percent of at least one active ingredient; according to claim 1 b) from about 20 percent to about 70 percent of water; c) from about 0.1 percent to about 10 percent of a suspending system; d) from about 0 percent to about 40 percent, e.g. from about 20 percent to about 40 percent of a sweetening agent; and e) from about 0 percent to about 0.2 percent of excipients. In that embodiment, based upon the total weight of active ingredient, from about 50 percent to about 75 percent of the active ingredient is in the immediate release dose portion and from about 25 percent to about 50 percent of the active ingredient is in the controlled release dose portion. In this same embodiment, based upon the total weight of the liquid suspension, from about 0.025 percent to about 30 percent of that dosage form is com prised of active ingredient in the immediate release dose portion and from about 0.0125 percent to about 0.025 percent of that dosage form is comprised of active ingredient in the controlled release dose portion.

In certain embodiments, the viscosity of the suspension of the present invention may range from about 400cps to about 1500 cps as measured by a Brookfield DV-I+ Viscometer using a # 31 spindle and speed of 12 rpm under temperature conditions of about 25 °C.

Another embodiment of the present invention is directed to an aqueous suspension dosage form containing active ingredient particles that are substantially covered or coated with one layer of the controlled release coating, and/or containing active ingredient particles that are dispersed in a matrix comprised of the controlled release composition.

The dosage forms of the present invention are intended to deliver an effective amount of active ingredient, in one or two daily adm inistrations. An "effective amount" of analgesic is one that provides relief from pain in a patient. For example, a typical adult dose of ibuprofen may range from about 2.9 to about 12 mg/kg weight of the patient given every 4 to 6 hours, for a typical daily dose ranging from about 11.6 to about 72 mg/kg/day. Therefore, administration of an effective amount of ibuprofen to a typical 70 kg adult may involve once or twice daily administration of about 5 ml to about 60 ml of the formulation of the present invention containing, for example, 40 mg/ml ibuprofen. A typical pediatric dose of ibuprofen may range from about 5 to about 10 mg/kg given every 4 to 6 hours, for a typical daily dose ranging from about 20 to about 60 mg/kg/day. Administration of an effective amount of ibuprofen to a typical 15 kg child may involve once or twice daily administration of about 5 ml to about 30 ml of the formulation of the present invention containing, for example, 20 mg/ml ibuprofen.

The oral administration of the dosage forms of the present invention provides the user with the active ingredients, in an optional immediate release dose as well as in a controlled release dose that continues to release the active ingredient from the dosage form after about 6 hours, e.g. after about 8 hours or after about 10 hours from administration. Beneficially, we have unexpectedly found how to effectively stabilize the release characteristics of the controlled release portion of the dosage form throughout the shelf life of the product and throughout the period of treatment, regardless of whether the dosage form is designed as a liquid dosage form, such as a suspension, or as a dry dosage form that can be reconstituted with water prior to administration. Specifically, we have overcome the challenge of preventing active ingredient release from the particles in the product prior to ingestion, while enabling controlled release of active ingredient from those same particles in the g.i. fluids.

Advantageously, the formulations of the present invention may be used in a variety of formats including, for example, (i) accurately-measurable single dose dry formulations or liquid suspensions; (ii) multi-dose granular formulations having significant dose flexibility obtainable by measuring different amount of granules to be resuspended on an as-needed basis; (iii) multi-dose liquid suspensions; and (iv) concentrated drops in which the active ingredient is suspended, which is particularly useful in pediatric applications.

In addition, since the formulation is convenient to administer and swallow, and the number of daily doses of active ingredient is reduced, the overall patient compliance is achieved. Additional benefits are anticipated in pediatric practice due to the ease of swallowing and administering.

Unlike prior art controlled release pharmaceutical suspensions, which require a series of enteric coatings to be applied to the pharmaceutical active agent in order to yield a shelf stable suspension, the suspended pharmaceutical particles of the present invention need only be coated with one layer of the novel sustained release coating in order to achieve stability in the presence of water or other water-miscible co-solvents.

The following examples further illustrate the invention, but are not meant to limit the invention in any way.

### Example 1: Preparation of Controlled Release Coating Solution

A coating solution was prepared by dispersing methacrylate co-polymer, which is commercially available from Rohm Pharma, Inc. under the tradename, "Eudragit L-100," and cellulose acetate in a solvent containing, based upon the total weight of the solvent, 98% acetone and 2% water under ambient conditions.

The resulting coating solution contained, based upon the total wet coating solution, 7.6% of cellulose acetate, 0.4% methacrylate co-polymer, 90.2% acetone, and 1.8% water.

The relative amounts of solids were, based upon the total weight percent of the dried coating solution, 95.00% of cellulose acetate and 5.00% methacrylate co-polymer.

### Example 2: Preparation Of Coated Active Ingredient

Preparation of Ibuprofen Pre-Mixture: Ibuprofen USP powder was combined with colloidal silicon dioxide to form the following ibuprofen pre-mixture:

| *Component* | *Weight Percent** |
|---|---|
| Colloidal silicon dioxide | 2.00% |
| Ibuprofen USP | 98.00% |

| | |
|---|---|
| *based upon total weight of Ibuprofen pre-mixture | |

Preparation of Coated Ibuprofen Granules: The ibuprofen mixture prepared above was then coated with the wet controlled release coating solution prepared in accordance with Example 1 at a rate of about 20.0 g/min in a Glatt GPCG-5/9 Wurster fluid bed coating unit under product temperature conditions of about 29-32°C. The resulting coated ibuprofen granules contained, based upon the total dry weight of the ibuprofen granules and the controlled release coating, about 20% of the controlled release coating.

### Example 3: Production Of The Suspension Base Containing Immediate Release Dose And Controlled Release Dose

Preparation of the Suspension Base

**Table A: Components of Suspension Base**

| Ingredients | Tradename | Percent (w/v) | mg/5mL |
|---|---|---|---|
| Purified Water, | | 50.0 | 2. |
| Pregelatinized | Ultrasperse | 1.5 | 0. |
| Xanthan Gum, | Xantural | 0.18 | 0. |
| Glycerin, USP | | 10.0 | 0. |
| Sucrose, NF | | 30.0 | 1. |
| Polysorbate 80 K | | 0.05 | 0. |
| Citric Acid, | | 0.18 | 0. |
| Sodium | | 0.20 | 0. |
| Purified Water, | | 22.4 | 0. |
| TOTAL | | 114.5 | 5. |

As indicated in Table A above, purified water USP was charged into a mixing tank equipped with a Scott Turbon high shear mixer and mixed at about 500 rpm to about 1000 rpm in order to create a good vortex. The pregelatinized starch and xanthan gun were then added to the mixing tank and mixed for 20 minutes. The glycerin was then added thereto and mixed for 5 minutes. The sucrose was then added thereto and mixed for 10 minutes. The polysorbate-80 NF, citric acid USP and sodium benzoate NF were added sequentially, and then the resulting mixture was mixed for 10 minutes. The remainder of the purified water was then added thereto with mixing to form the suspension base.

### Preparation of Suspension with Active Ingredients:

After 2000.0 mg of Ibuprofen USP was sieved through a 50 mesh screen, 25.0 mL of the suspension base prepared above was added thereto with mixing until the mixture was homogeneous.

1276 mg of the controlled release coated ibuprofen prepared in accordance with Example 2 (which contained 78.4% of active ibuprofen) was then sieved between 60 and 80 mesh screens and then added to the mixture.

The resulting suspension was then diluted to 100.0 mL volume with additional suspension base and mixed until the resulting suspension was homogeneous. After sieving the resulting suspension through a 40 mesh screen, the resulting final sieved suspension contained 100 mg/5mL of the immediate release ibuprofen dose and 50mg/5mL of the controlled release ibuprofen dose. The relative amounts of ibuprofen particles were, based upon the total dose of the final sieved suspension:

| | |
|---|---|
| Ibuprofen USP (Immediate Release Dose) | ................100.0 mg/5 mL |
| Coated Ibuprofen (Controlled Release Dose) | ...............50.0 mg/5 mL |

### Example 4: Dissolution Analysis Of Suspension Base Containing Immediate Release Dose And Controlled Release Dose

900 mL of a pH 5.6 acetate buffer dissolution media was placed in each of the three containers of a USP Type II apparatus with paddles. A 5.0 mL sample of the final suspension produced in Example 3 was then independently added to each of the three containers and mixed at a speed of 50 r.p.m at 37°C until the mixture was homogeneous.

After 0.5, 1, 2, 3, 4, 6, 8, and 12 hours, respectively, thereafter, 10 ml samples of the suspension/buffer mixture were independently removed from the containers.

Each 10 ml sample was then independently analyzed for ibuprofen content using a high pressure liquid chromatograph (HPLC) equipped with a Waters^{®} 717 Autoinjector and a Waters^{®} 486 UV detector set at a wavelength of 254 nm in order to derive dissolution curves for the ibuprofen at 0.5, 1, 2, 3, 4, 6 , 8, and 12 hours, respectively. Each of the samples was compared to a standard ibuprofen sample containing 0.167 mg ibuprofen/mL acetate buffer (pH 5.6) dissolution media, which correlated to the theoretical concentration required for 100% release of ibuprofen.

The mobile phase used in the HPLC was prepared using a sample containing 55% acetonitrile and 45% of 18mM potassium phosphate buffer. The injection volume was 200 µL with a run time of approximately 7 minutes and a pump flow of 1.5 mL/min. The column used for analysis was a Phenomenex LUNA^{®}5um C8 (4.6 mm X 15 cm).

The results of this dissolution analysis are set forth in FIG. 1, which indicated that the suspension of the present invention could contain both an immediate release dose of an active ingredient as well as a controlled release dose of an active ingredient whereby the controlled release dose released ibuprofen over a period of about 12 hours from initial administration.

### Example 5: Production Of The Suspension Base

### Containing Controlled Release Dose

The procedure of Example 3 was repeated, but without the addition of the 2000.0 mg of Ibuprofen USP.

The resulting final sieved suspension contained 1 00mg/5mL of the controlled release ibuprofen dose.

### Example 6: Dissolution Analysis Of Suspension Base

### Containing Controlled Release Dose

The procedure of Example 4 was repeated, but with samples of the final suspension produced in Example 5.

The results of the dissolution analysis are set forth in FIG. 2,) which indicated that the suspension of the present invention could contain a controlled release dose of an active ingredient in the substantial absence of an immediate release dose of an active ingredient, whereby the controlled release dose released ibuprofen over a period of about 12 hours from initial administration.

## Claims

1. A pharmaceutical liquid suspension dosage form comprising:
a) particles of an NSAID, said particles being substantially covered with one layer of a controlled release composition, and
b) a vehicle for the administration of the particles comprising water,
wherein the pharmaceutical liquid suspension dosage form has a duration of therapeutic effect for at least about 8 hours after its initial administration to a mammal,
wherein the controlled release composition is comprised of, based upon the total dry weight of the coating, from greater than 0 percent and less than 90 percent of an insoluble film forming polymer and from greater than 0 percent to less than 10 percent of an enteric polymer, wherein the weight ratio of the insoluble film forming polymer and the enteric polymer in the controlled release coating is from 80:20 to 99: 1.
wherein the NSAID is ibuprofen, 2-(4-isobutylphenyl) propionic acid, or a pharmaceutically acceptable salt thereof,
said particles are comprised of a core that is substantially covered by the controlled release composition, and
the suspension comprises an effective amount of a buffering agent in order to have the pKa of said NSAID contained in the controlled release portion of the liquid suspension dosage form be greater than the pH of the overall liquid suspension dosage form.

2. The dosage form of claim 1, comprising a vehicle for the administration of the particles, wherein the vehicle comprises water or a mixture of water and a glycol, alcohol or glycerol as a pharmaceutically acceptable water-miscible co-solvent.

3. The dosage form of claim 1, wherein the insoluble film forming polymer is cellulose acetate, ethylcellulose, poly(ethyl acrylate, methyl methacrylate, trimethylammonioethyl methacrylate chloride) in a 1:2:0.1 weight ratio or a copolymer or mixture thereof.

4. The dosage form of any one of claims 1 to 3, wherein the enteric polymer is hydroxypropyl methylcellulose phthalate, hydroxypropyl methylcellulose acetate succinate, cellulose acetate phthalate, polyvinylacetate phthalate, a polymethacrylate-based polymer or a copolymer or mixtures thereof.

5. The dosage form of claim 4, wherein the polymethacrylate-based polymer is poly(methacrylic acid, methyl methacrylate) in a weight ratio of 1:2 and/or poly(methacrylic acid, methyl methacrylate) in a weight ratio of 1:1.

6. The dosage form of any one of claims 1 to 5, wherein said controlled release coating contains, based upon the total weight of the controlled release composition, less than 1 percent of enteric polymer.

7. The dosage form of any one of claims 1 to 5, for use in treating pain in a mammal.

## Patentansprüche

1. Pharmazeutische Flüssigsuspensionsdosierungsform, umfassend:
a) Partikel eines nicht-steroidalen Antirheumatikums, wobei diese Partikel im Wesentlichen mit einer Schicht einer Zusammensetzung für eine kontrollierte Freisetzung überzogen sind, und
b) ein Vehikel für die Verabreichung der Partikel, welches Wasser umfasst, wobei die pharmazeutische Flüssigsuspensionsdosierungsform eine therapeutische Wirkung von mindestens etwa 8 Stunden Dauer nach ihrer ursprünglichen Verabreichung an ein Säugetier hat,
wobei die Zusammensetzung für eine kontrollierte Freisetzung, basierend auf dem Gesamttrockengewicht des Überzugs, mehr als 0 Prozent und weniger als 90 Prozent eines einen unlöslichen Film bildenden Polymers und mehr als 0 Prozent bis weniger als 10 Prozent eines magensaftresistenten Polymers umfasst, wobei das Gewichtsverhältnis des einen unlöslichen Film bildenden Polymers zum magensaftresistenten Polymer im Überzug für eine kontrollierte Freisetzung 80:20 bis 99:1 beträgt,
wobei es sich bei dem nicht-steroidalen Antirheumatikum um Ibuprofen [2-(4-Isobutylphenyl)propionsäure] oder ein pharmazeutisch unbedenkliches Salz davon handelt,
wobei die Partikel aus einem Kern bestehen, der im Wesentlichen mit der Zusammensetzung für eine kontrollierte Freisetzung überzogen ist, und
die Suspension eine wirksame Menge eines Pufferungsmittels umfasst, so dass der pKa des in dem Teil mit kontrollierter Freisetzung der Flüssigsuspensionsdosierungsform enthaltenen nicht-steroidalen Antirheumatikums größer ist als der pH-Wert der gesamten Flüssigsuspensionsdosierungsform.

2. Dosierungsform nach Anspruch 1, welche ein Vehikel für die Verabreichung der Partikel umfasst, wobei das Vehikel Wasser oder eine Mischung von Wasser und einem Glykol, Alkohol oder Glycerin als pharmazeutisch unbedenkliches, wassermischbares Kosolvenz umfasst.

3. Dosierungsform nach Anspruch 1, wobei es sich bei dem den unlöslichen Film bildenden Polymer um Celluloseacetat, Ethylcellulose, Poly(ethylacrylat, methylmethacrylat, trimethylammonioethylmethacrylatchlorid) in einem Gewichtsverhältnis von 1:2:0,1 oder ein Copolymer oder eine Mischung davon handelt.

4. Dosierungsform nach einem der Ansprüche 1 bis 3, wobei es sich bei dem magensaftresistenten Polymer um Hydroxypropylmethylcellulosephthalat, Hydroxypropylmethylcelluloseacetatsuccinat, Celluloseacetatphthalat, Polyvinylacetatphthalat, ein Polymer auf Polymethacrylatbasis oder ein Copolymer oder eine Mischung davon handelt.

5. Dosierungsform nach Anspruch 4, wobei es sich bei dem Polymer auf Polymethacrylatbasis um Poly(methacrylsäure, methylmethacrylat) in einem Gewichtsverhältnis von 1:2 und/oder Poly(methacrylsäure, methylmethacrylat) in einem Gewichtsverhältnis von 1:1 handelt.

6. Dosierungsform nach einem der Ansprüche 1 bis 5, wobei die Beschichtung für eine kontrollierte Freisetzung, basierend auf dem Gesamtgewicht der Zusammensetzung für eine kontrollierte Freisetzung, weniger als 1 Prozent magensaftresistente Polymere enthält.

7. Dosierungsform nach einem der Ansprüche 1 bis 5 zur Verwendung bei der Behandlung von Schmerzen in einem Säugetier.

## Revendications

1. Forme pharmaceutique de suspension liquide pharmaceutique comprenant :
a) des particules d'un NSAID, lesdites particules étant sensiblement recouvertes avec une couche d'une composition à libération contrôlée, et
b) un véhicule pour l'administration des particules comprenant de l'eau, la forme pharmaceutique de suspension liquide pharmaceutique a une durée d'effet thérapeutique pendant au moins environ 8 heures après son administration initiale à un mammifère,
la composition à libération contrôlée est constituée de, sur la base du poids sec total de l'enrobage, de plus de 0 pour cent et moins de 90 pour cent d'un polymère filmogène insoluble et de plus de 0 pour cent à moins de 10 pour cent d'un polymère entérique, le rapport en poids du polymère filmogène insoluble et du polymère entérique dans l'enrobage à libération contrôlée est de 80:20 à 99:1,
le NSAID étant l'ibuprofène, l'acide 2-(4-isobutylphényl)propionique, ou un sel pharmaceutiquement acceptable de celui-ci,
lesdites particules sont constituées d'un noyau qui est sensiblement recouvert par composition à libération contrôlée, et
la suspension comprend une quantité efficace d'un agent tampon afin que le pKa dudit NSAID contenu dans la partie à libération contrôlée de la forme pharmaceutique de suspension liquide soit supérieur au pH de la forme pharmaceutique de suspension liquide totale.

2. Forme pharmaceutique de la revendication 1, comprenant un véhicule pour l'administration des particules, le véhicule comprenant de l'eau ou un mélange d'eau et d'un alcool de glycol ou de glycérol en tant que co-solvant miscible avec l'eau pharmaceutiquement acceptable.

3. Forme pharmaceutique de la revendication 1, dans laquelle le polymère filmogène insoluble est l'acétate de cellulose, l'éthylcellulose, le poly(acrylate d'éthyle, méthacrylate de méthyle, chlorure de méthacrylate de triméthylammonioéthyle) dans un rapport en poids 1:2:0,1 ou un copolymère ou mélange de ceux-ci.

4. Forme pharmaceutique de l'une quelconque des revendications 1 à 3, dans laquelle le polymère entérique est le phtalate d'hydroxypropylméthylcellulose, l'acétate-succinate d'hydroxypropylméthylcellulose, l'acétate-phtalate de cellulose, le phtalate de poly(acétate de vinyle), un polymère à base de polyméthacrylate ou un composant ou des mélanges de ceux-ci.

5. Forme pharmaceutique de la revendication 4, dans laquelle le polymère à base de polyméthacrylate est le poly(acide méthacrylique, méthacrylate de méthyle) dans un rapport en poids de 1:2 et/ou le poly(acide méthacrylique, méthacrylate de méthyle) dans un rapport en poids de 1:1.

6. Forme pharmaceutique de l'une quelconque des revendications 1 à 5, dans laquelle ledit enrobage à libération contrôlée contient, sur la base du poids total de la composition à libération contrôlée, moins de 1 pour cent de polymères entériques.

7. Forme pharmaceutique de l'une quelconque des revendications 1 à 5, pour utilisation dans le traitement de la douleur chez un mammifère.
